# EUROPEAN PATENT SPECIFICATION

(11) **EP 2 880 450 B1**
(45) Date of publication and mention of the grant of the patent: **29.11.2017**
(21) Application number: 13742213.5
(22) Date of filing: 25.07.2013
(51) Int. Cl.: G01N 33/68

(54) **DETECTION OF MIXTURES IN MASS-SPECTROMETRIC MICROBE IDENTIFICATION**
DETEKTION VON MISCHUNGEN BEI DER MIKROBENDETEKTION DURCH MASSENSPEKTROMETRIE
DÉTECTION DES MÉLANGES À IDENTIFICATION DE MICROBES PAR SPECTROMÉTRIE DE MASSE

(30) Priority: 03.08.2012 DE 102012015446
(43) Date of publication of application: 10.06.2015
(73) Proprietor: Bruker Daltonik GmbH, 28359 Bremen (DE)
(72) Inventor: FRANZEN, Jochen, 28357 Bremen (DE); KLEPEL, Stefan, Taucha 04425 (DE); KOSTRZEWA, Markus, 28865 Lilienthal (DE); MAIER, Thomas, 28865 Lilienthal (DE)
(86) International application number: PCT/EP2013/065764
(87) International publication number: WO 2014/019943

(56) References cited:
- EP-A1- 1 437 673
- EP-A1- 2 354 796
- GB-A- 2 467 636
- US-A1- 2011 224 104

## Description

The invention relates to the identification of microbes in a sample by calculating the similarities between a mass spectrum of the sample and reference spectra in spectral libraries; it particularly concerns the detection of microbe mixtures.

### Prior Art

The routine, fast and error-free identification of many samples of microorganisms plays an important role particularly in clinical and extra-clinical infection diagnostics, in hygiene monitoring in hospitals or rivers and lakes used for swimming, in food analysis, in the monitoring and control of biotechnological processes and in microbiological research. The term "microorganisms", here called "microbes" for short, describes all microscopically small organisms, for example unicellular fungi (e.g. yeasts), algae, or protozoa (e.g. plasmodia as malaria pathogens). The identification focuses on bacteria, however.

The identification of microbes basically means determining their species and thus categorizing them in the taxonomic hierarchy, which ranges from the topmost level, the domain (bacteria, archaea and eukaryotes), right down to order, family, genus and species.

The practice of classifying microorganisms into species originates from a time when the taxonomy of microbes was based largely on distinguishing them by means of biochemical reactions. It is imprecise in many cases and often does not classify by uniform genetic relationship. The conventional biological definition for distinguishing species from each other is the unlimited ability for sexual reproduction between members of the same species, but not between members of different species. Naturally this definition cannot be applied to microorganisms, and therefore the classification of species is often arbitrary. Modern methods of molecular biology have corrected many assignments of species to a genus, family or even an order, have combined many species and defined new species. In the case of bacteria, further taxonomic classes, subspecies, were introduced below the species. Furthermore, observations in medicine and cell biology have led to the inclusion of serovars or sero-types, which are particularly distinguished by different types of attachment behavior on cell membranes, but do not constitute a separate species or subspecies.

The many new definitions and re-assignments of species to genera and families have resulted in as many new names for microbes, but these have not always become established in routine daily practice. Consequently, the degree of relationship between microbes is not always recognizable from the usual, and sometimes outdated, names. Some findings on re-assignments and more correct relationship structures have yet to be generally accepted. A knowledgeable specialist is required to determine these relationships.

In contrast to the traditional identification of microorganisms, new molecular-biological identification methods have been known for some years. These are based on, for example, DNA or RNA sequence analysis after amplification of specified gene segments by polymerase chain reaction (PCR), or mass-spectrometric detection of specific molecular cell components of microorganisms. These new methods are far superior to conventional methods in terms of specificity (true-negative rate), sensitivity (true-positive rate), other error rates and analytical speed.

The identification of a microbe sample by mass-spectrometric measurement, as defined in this document, usually entails determining the species, represented in effect by the name of this species, as is common practice in microbiological laboratories or in clinical applications. In some cases it is only possible to determine the genus, represented by the commonly used genus name. Only occasionally is it possible to determine the subspecies or - in favorable cases - the serotype or the strain. In a wider sense, identification can also mean characterization in terms of other characteristics, such as the pathogenicity of a microorganism (ability to cause disease), or the resistance of a microorganism to antibiotics, but these types of identification are not the issue here.

The identification of bacteria by mass-spectrometric measurements has been described in detail in the review by van Baar (FEMS Microbiology Reviews, 24, 2000, 193-219: "Characterization of bacteria by matrix-assisted laser desorption/ionization and electrospray mass spectrometry"), for example. The identification is achieved by means of a similarity analysis between a mass spectrum of the bacteria to be identified and reference mass spectra (simply called reference spectra in the following) of known bacteria. During the similarity analysis, a similarity indicator is assigned to each of the reference spectra, characterizing the degree of agreement between the reference spectrum and the mass spectrum of the sample. A bacterium can be regarded as identified if, for example, the similarity indicator is significantly higher than the similarity indicators for all other reference spectra or higher than a specified minimum score. In the following, the similarity indicators are also referred to as "similarity scores" or simply "similarities".

The reference spectra are generally collated in one or more collections, for which the term "library" is used in the following. A library thus comprises one or more collections of reference spectra. For applications in medical diagnostics, such libraries of reference spectra must be validated according to legal stipulations. Validation of a library of reference spectra requires every entry to be traceable and very accurately documented. The reference spectra are obtained from precisely characterized strains.

Living microorganisms are collected worldwide in many institutes in the form of frozen or freeze-dried strains. The term "strain" describes a population which has been multiplied from a single organism and identified with recognized certainty in a specialized laboratory. Since the microbes are collected and stored in different places around the world, there are also many strains worldwide which belong to the same species or subspecies. Although these strains are classified as the same species or subspecies, there show sometimes slightly different mass spectra, indicating that there are individual differences (as is the case with animals or plants of the same species). The strains bear internationally agreed designations after the name of the species or subspecies.

As a rule, a library of reference spectra contains not just one reference spectrum for a microbial species (or subspecies), but several reference spectra from different strains, because the mass spectra of the strains differ slightly. For entering a new reference spectrum into a library, it is often crucial that it differs from the existing reference spectra of this species in order to clearly map the breadth of variation of the mass spectra of this species or subspecies. Identical reference spectra for microbes of a particular species, even if they originate from different strains, do not contribute to a better identification. At least five, even better ten, fifteen or more reference spectra should be available for one microbe species, especially if the mass spectra of the strains differ significantly from each other. There are, however, cases where only one or two reference spectra are contained in the library because there is a lack of strains which have been identified with certainty.

The generation of mass spectra of the microbes usually starts with a cleanly isolated colony (an "isolate") on a solid, usually gelatinous nutrient medium. A small swab, for example a hygienically clean wooden tooth pick, is used to transfer a tiny quantity of microbes, in routine operation, from the selected colony to the mass-spectrometric sample support. There the sample is cell disrupted in the known way; matrix substance is added, and the sample is analyzed in a time-of-flight mass spectrometer with ionization by matrix-assisted laser desorption. The ions from each pulse of laser light result in an individual mass spectrum, which is, however, statistically unreliable and often has relatively large amounts of noise. In order to obtain reliable and noiseless mass spectra, several hundred of these individual mass spectra are added to form a sum mass spectrum. The terms "mass spectrum of a microbe" or simply "microbe spectrum" shall always denote this sum mass spectrum.

The mass spectrum of a microbe isolate is the abundance profile of the mass values of the ions of the soluble microbe material. From the ten thousands of substances inside a microbe cell, only the around 50 to 200 most abundant (or easiest to ionize) components are measured above the measurement threshold. The ions here are very predominantly protein ions, and particularly ions of the different types of proteins which are contained in the ribosomes. A ribosome always contains the same number of around 40 to 60 identical species-specific proteins. Each microbe cell contains far more than 10,000 completely identical ribosomes, i.e. more than 10,000 molecules of the species-specific proteins in each case. As rule, these ribosomal proteins belong to the most prominent peaks of the mass spectrum. The profile of the proteins which this microbe spectrum reproduces is very characteristic of the species of microbe in question because every species produces its own, genetically predetermined proteins, each having their own characteristic masses. Similarly, the abundances of the individual proteins in the microbes, in as much as they can be measured by mass spectrometry, are also predetermined either genetically or, as with the ribosomes, by the structure of the cell. The nutrient medium and the degree of maturity of the colony are almost irrelevant here. The identifications are therefore extremely stable and hardly depend at all on the type or duration of the culturing or on the sample preparation. The protein profiles are characteristic of the microbes in rather the same way that fingerprints are characteristic of humans. In the mass spectra, the most useful information for identifications is found in the mass range from around 3,000 daltons to 15,000 daltons.

Despite this stability, it has proved advantageous to standardize the culturing and sample preparation as much as possible. Reference spectra for spectral libraries are generated by producing colonies of microbes of specific, accurately documented strains and acquiring mass spectra from them. For statistical reasons many sum mass spectra are always acquired and evaluated for one reference spectrum. The sum mass spectra of microbes usually contain around 50 to 200 mass signals which are clearly separated, usually by empty spaces in the mass spectra, although many of them are pure noise because the search for mass signals is set to very high sensitivity. The reference spectra are therefore usually reduced to a maximum number of 70 or 100 mass signals, for example, by removing the noise signals and the signals whose intensity is too low. Specialists even consider a limit of 50 mass signals to be sufficient. The information content of a mass spectrum with 50 mass signals in the mass range between 3,000 and 15,000 daltons, where far more than 2,000 distinguishable mass signals can occur even at reduced mass resolving power, is incredibly high, even without taking account of the intensity differences (theoretically more than 2000⁵⁰ ≈ 10¹⁶⁵ patterns can be differentiated from each other).

The mass spectra of the microbes to be identified, called "sample spectra" below for short, are generated in a similar way from repeat spectra and limited to a predetermined number of mass signals in order to exclude noise signals as far as possible. The number of mass signals in these sample spectra is usually selected to be slightly higher than the number of mass signals in the reference spectra.

The identification of the microbes is based on similarity analyses of the sample spectra and the reference spectra in a library. There are various types of similarity analysis, which are usually based on different forms of reference spectra. Several types of identification algorithm and reference libraries have become known in the literature, but they will not be dealt with any further here. The similarities between two mass spectra are usually characterized by a "similarity indicator" (in the following also called "similarity score" or simply "similarity"). The applicant's company has developed a very simple and fast mass-spectrometric identification method which has an extraordinarily high success rate, as verified by numerous studies by independent research groups. Since the intensities of the mass signals vary greatly, the similarity indicator is essentially based on the masses of the mass signals; the intensities are taken into account only slightly.

The above-mentioned identification method is based on a special computational method for the similarity indicators. Since similarity indicators are very important below, their computation as we perform it in our company is briefly explained here. The computational method is based on three partial measures. A first partial measure of the similarity indicator is represented by the number of mass signals in the microbe spectrum and reference spectrum ("number of hits") matching within a mass tolerance interval, divided by the number of mass signals in the reference spectrum, but with all the mass signals being weighted pro rata with their presence. The presence is the percentage for the occurrence of this mass in the reference spectra for repeat measurements. The mass tolerance interval can be given as an absolute figure in daltons or as a relative value in ppm (parts per million). A second partial measure results from the number of hits divided by the number of mass signals in the microbe spectrum, again weighted pro rata with the presences in the reference spectrum. The first and second partial measures can each have a value of up to 1.00. The third partial measure is calculated from the similarity between the respective intensities of the mass signals which match, the presences again being taken into account in a multiplicative way. This third partial measure is normalized for all the mass signals so that when all intensities are equal the partial measure has the value 1.00.

These three partial measures are now simply multiplied together and thus give the indicator for the similarity between reference and microbe spectrum. Since each of the three partial measures can have a value up to 1.00, the maximum value of the similarity indicator is likewise 1.00. Hundred thousands of verified identifications made by this type of similarity calculation have now shown that reliable identification is practically always linked to a similarity score greater than 0.10. In order to derive numbers that are easier to handle, a transformation can be undertaken by multiplying by 1000 and then taking the logarithmic value, which results in a maximum similarity indicator of S = 3.00 for identical spectra, and a minimum similarity indicator of S = 2.00 required for the identification of a species. This transformation is not essential, but it does have a high psychological value; any other transformations can also be applied if they prove to be more practicable.

The calculation of these similarity indicators can be implemented in a very fast algorithm. The computers used with mass spectrometers today require only a few milliseconds for scale adjustments and computations of the similarity indicator.

The results are usually presented in a "score list", at least as an interim result, in which the most similar reference spectra are given, ordered according to the similarity indicators, together with the names of the corresponding microbe species, subspecies and strains. Thousands of verified identifications have shown that, with a certainty usually far in excess of 95 %, similarity indicators between *S*_{f} = 1.70 and *S*_{g} = 2.00 identify at least the family and probably the genus, while indicators between *S*_{g} = 2.00 and *S*ₛ = 2.30 definitely identify the genus and probably the species. Similarity indicators above *S*ₛ = 2.30 identify the individual species with a high level of certainty. The limits stated here are surprisingly correct throughout bacterial classification; they have been confirmed in many large-scale studies by different research groups. These similarity scores are used in the most common mass-spectrometric identification system, "MALDI Biotyper®" from Bruker Daltonics. For other systems, conversions must be carried out where necessary.

As has already been indicated briefly, in the development history of this mass-spectrometric identification method, it has become established practice to create a tabular score list with the names of the microbe strains whose reference spectra resulted in the best similarity scores as an intermediate result of the identification. This list is sorted in the order of the similarity to the sample spectrum. All reference spectra with a similarity above a score of S = 1.50 are displayed in the form of a table, for example; if there are more than 30 most similar reference spectra, the table can be cut short. Without using any similarity threshold, the score list often contains simply the 20 to 30 most similar reference spectra. The table can also be cut short if a large jump occurs in the similarity scores, a jump of *ΔS* = 0.60, for example. This score list should then be assessed by a specialist, using his/her experience to make the definitive identification. The specialist must in particular assess whether the sample is a mixture of different microbe species when several microbe species are listed in the score list.

Since the mass-spectrometric identification method is nowadays used in hundreds of routine laboratories, the requirement to have a specialist assessment is no longer appropriate. Methods are increasingly being demanded which run automatically as far as possible, but nevertheless have a high identification certainty also when microbe mixtures are present. The assessment of the score list therefore has to be largely transferred to a computer program; only in rare cases may a result remain undetermined.

The identification of microbes from cleanly separated colonies on an agar surface ends with an unequivocal identification in most cases. But cleanly separated colonies on an agar are by no means a guarantee that mixtures of microbes are not present after all. There are microbes which are mobile and spread in an often invisible film over the surface of the agar within the culture period. They may avoid another colony if the excreted metabolic waste products of the foreign colony do not appeal to them; but if the metabolic waste products are appealing, the migrating microbes can also collect there and thus form a mixture with the microbes of the local colony. The migrated microbes can even overgrow the microbes of the local colony in terms of numbers.

The microbe samples can also originate from a culture in a liquid, however; a blood culture, for example. After purification and separation by centrifugation, the microbe samples are then usually present in pellet form. In more than 70% of these cases, there is no mixture of microbes here either, because often only one species is present or one species has extensively overgrown all other species of microbes. There are exceptions if two species tolerate each other or even live together symbiotically. The probability that the sample contains a mixture of two or even several microbe species is therefore certainly in the order of a double digit percentage.

A mixture can be suspected when there are reference spectra in the first 20 or 30 best hits of the score list which belong to at least two different microbe species. If a mixture is suspected, methods for the identification of microbes in mixtures can be used, as described in the documents DE 10 2009 007 266 A1 (M. Kostrzewa et al., corresponding to GB 2 467 636 A or US-2010-0248298-A1). These documents are included here by reference. The documents describe a method for the identification of microbe species in mixtures which is designated below as "mixture analysis by combination spectra". In this method for the determination of microbe species in mixtures, all possible combination spectra, each comprising two mass spectra from the score list, which do not belong to the same microbe species are generated. If the similarity score of one of these combination spectra in relation to the sample spectrum is greater than any of the similarity scores of the individual spectra used, then it is quite probable, but by no means certain, that the sample is a mixture of the microbe species involved in the most similar combination spectra. If there is a degree of certainty that the sample is a mixture, the two (or more) microbe species whose spectra resulted in the best combination can be deemed to be components of the mixture.

This method has a self-confirming character, however, which can lead one to believe that a more similar combination spectrum always corresponds to a mixture. But it can also happen that the score list contains reference spectra of two microbe species without the sample being a mixture. Closely related microbe species occasionally have mass spectra which are so similar that reference spectra of the second microbe species must inevitably appear among the best similarity hits. If combination spectra are generated here and compared with the sample spectrum, it can be expected with near certainty that at least one of the combination spectra provides a better similarity score, thus giving the false impression that a mixture is present.

The document DE 10 2009 007 266 A1 cited above for the determination of the components of a mixture therefore recommends that the degree of relationship between the different types of microbe be determined. If there is a close relationship, one can assume that the sample is not a mixture. For the reasons stated above, the names often do not mirror the degree of relationship, and so an experienced specialist is again required here to carry out this task. Such specialists are rarely found in routine laboratories. When undertaking a visual evaluation of the interim results, it is easy for less experienced laboratory staff to wrongly assume that the sample is a mixture.

For mass spectrometric identifications of microbes, there is a need for a method which can recognize automatically, as secure as possible, whether the sample is a mixture of microbe species or not.

When the term "score list" is used in the following, this means the reference spectra most similar to the sample spectrum and the designations of the associated microbe strains and microbe species, even if these are not in the form of a list, but only in the form of a collection of data in the computer memory, for example. The most similar reference spectra can be differentiated from all other reference spectra in a library by a specified similarity threshold, or by a specified number of the most similar reference spectra, or by a difference between the similarity scores and the next most similar reference spectrum exceeding a specified value. A microbe species can be represented in the score list with only one single reference spectrum (if, for example, only one reference spectrum of this microbe species is present) or with ten or 15, if so many reference spectra of strains of this microbe species are actually present in the library and are all correspondingly similar to the sample spectrum.

### Objective of the Invention

The objective of the invention is to provide methods for the recognition of mixtures of two or more microbe species and for the identification of the microbe species concerned, when carrying out a mass-spectrometric identification of microbe samples.

### Summary of the Invention

The method for the identification of microbe species in mixtures which is designated as "mixture analysis by combination spectra" recommends first an investigation whether the different microbe species in the score list are closely related with each other if the score list contains reference spectra of several microbe species. It is suggested in the documents that one should assume that a mixture is not present if the different microbe species in the score list are closely related.

This relationship often cannot be deduced from the names of the microbes. The investigation therefore requires an experienced specialist, and usually cannot be carried out regularly in a routine laboratory. For this reason, a first embodiment of the invention proposes having an experienced specialist determine the relationships between the microbes of all the reference spectra in the library once, and adding to the library a matrix-like relationship list with the microbe species names used for the reference spectra. This list is called an "exclusion list" here, because it indicates that a mixture is excluded. The exclusion list represents the relationship between microbe strains which have very similar mass spectra and are assigned to different microbe species. The relationship may be described by numbers r, with r = 1.0 for closely related strains of the same species, and r = 0.0 indicating no relationship at all. With the aid of this list, the computer program for the evaluation of the mass spectra can then determine whether such a near relationship exists, and negate the presence of a mixture when this is the case. It has been found that this exclusion list must not simply represent the relationship between microbe species, but rather the relationships between the strains of one microbe species and strains of other microbe species, in a complex way.

However, this method has the principal disadvantage that the list of relationships must always be updated and expanded when the reference library is extended. Since it should remain possible in principle to add further reference spectra to the library in the routine laboratory also, the specialist is always required in order to keep this list of relationships complete.

A second embodiment of the invention is based on the finding that what matters is not the taxonomically determined relationship between the microbes, but only the degree of similarity between their reference spectra. If the reference spectra of two strains of different microbe species are very similar (whether they are classed as related or not), reference spectra of both microbe strains must inevitably appear in the score list when one of the two microbe strains is present, provided that the similarity is sufficiently large. If a reference spectrum of one of the two microbe strains provides good similarity scores in relation to the sample spectrum, the second microbe strain must also have reference spectra with relatively high similarity.

To answer the question as to whether a sample is a mixture or not when there are two or more microbe species in the score list, a second embodiment of the invention therefore proposes to investigate the reference spectra of the different microbe species in the score list in respect of their mutual similarity, for example by means of the evaluation program for assessing the score list itself. This can be done using various mathematical methods, but the simplest way is to use the same similarity calculation as used for the identifications. If they are very similar, with similarity scores above an experimentally determined similarity threshold, no mixture is present. If, on the other hand, the reference spectra of the different microbe species are not similar to each other, this indicates to the user that the sample must be a mixture. Then the method of mixture analysis with combination spectra described above can be carried out in order to identify the microbes which make up the mixture.

In a third embodiment, if several microbe species are contained in the score list, the method of "mixture analysis with combination spectra" according to DE 10 2009 007 266 A1, GB 2 467 636 A, or US-2010-0248298-A1 is always carried out, but without already assuming firmly the presence of a mixture. The computer program goes through the score list, starting from the most similar reference spectrum. The most similar reference spectra usually all belong to one microbe species when reference spectra of several strains of this microbe species are present in the library. If the program now hits on a second microbe species, combination spectra are formed with all the reference spectra of the first microbe species. These combination spectra are then investigated regarding their similarity to the sample spectrum. The same method is applied for further reference spectra of the second microbe species, and also for reference spectra of a third microbe species if one is present. If combination spectra are obtained whose similarities to the sample spectrum are greater than the similarities of all the reference spectra in the score list, a mixture may be present. For the definitive decision on whether a microbe mixture is present, the similarity of the pairs of reference spectra from which the most similar combinations have been generated in each case are checked against each other. If the similarities are sufficiently low, below an experimentally determined threshold, there is a high probability that a mixture is present and this is indicated to the user. The microbes of those reference spectra which have led to the best combination spectra are deemed to be the components of the mixture. The degree of similarity of the two reference spectra of a combination even allows a statement to be made on the level of probability that a mixture is present.

### Illustrations

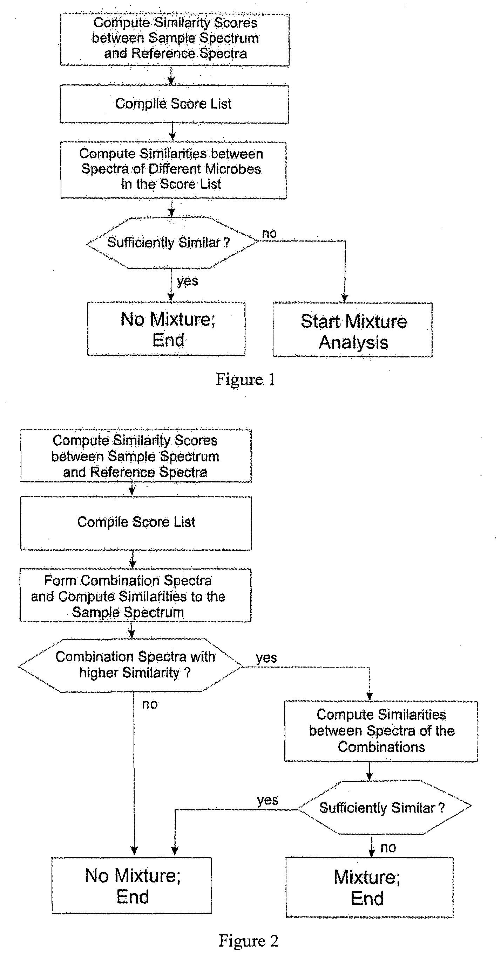

Figure 1 depicts a process diagram of the second embodiment of the mixture detection.
Figure 2 shows a process diagram for the third embodiment of the mixture detection.

### Embodiments

The invention proposes methods for the recognition of microbe mixtures which can be used in the mass-spectrometric identification procedure of a microbe sample by computing indicators for the similarity between the mass spectrum of the microbe sample (the "sample spectrum") and reference spectra. This identification method always involves first determining the most similar reference spectra and entering them into a score list. The score list may contain two or more microbe species which either constitute a mixture or originate from very closely related microbes.

A first embodiment of a method according to the invention for the detection of microbe mixtures in the mass-spectrometric identification of a microbe sample with compilation of a score list is characterized by the determination of the degrees of relationship between the microbe strains with the aid of an exclusion list, if two or more microbe species are present in the score list. The degrees of relationship may be described by numbers r, e.g., with r = 1.0 for closely related strains of the same species, *r* = 0.8 for closely related species of the same genus, *r* = 0.6 for closely related genera of the same family, r = 0.0 indicating no relationship at all. The microbe sample is indicated as a microbe mixture if the relationship is sufficiently low, with a degree of relationship r below a specified value. The method of mixture analysis with combination spectra described above can then be carried out in order to identify the microbes which make up the mixture.

The exclusion list must be drawn up by a specialist. The specialist can limit the investigation of the degrees of relationship to those cases where different microbe strains from different microbe species have very similar mass spectra. These microbe strains can be picked out automatically by an appropriate computer program by comparing all reference spectra with all others. The specialist then checks only this list of all those pairs of microbe strains whose mass spectra are very similar to each other.

It has been found to be imperative to take into account the relationships between individual strains of one microbe species and all strains of another microbe species, not simply the relationship between individual microbe species themselves, which makes the exclusion list very complex. For example, some strains of E. coli are closely related to strains of microbes of the genus Shigella, whereas other strains of E. coli are less closely related. Both belong to the family of enterobacteriaceae. Shigella cause bacillary dysentery or shigellosis, but most subspecies of E. coli are useful members of our intestinal flora; a correct identification is therefore of crucial diagnostic importance. We take the liberty of noting here that some microbiologists nowadays hold the view that Shigella are definitely not a genus in their own right, with four microbe species, nor even form an independent species, but are really only subspecies of E. coli. This view is also supported by the finding that there are further subspecies of E. coli which produce the same toxin as the four microbe species from the genus Shigella. This example shows that the specialist really is required to determine relationships and compile an exclusion list. It furthermore shows that the taxonomy of microbes is still in a state of continuous change and requires further reassignments.

The exclusion list allows the computer program to determine the degree of relationship, even if this cannot be recognized from the names of the microbe species being compared, or is even different for different strains of one microbe species in relation to strains of another microbe species. This list must, however, be constantly updated when further reference spectra are added to the library. This is a slight disadvantage if such an expansion of the reference library is carried out not by the manufacturer but at a routine, laboratory which does not have an appropriate specialist available. Even large libraries of reference spectra cannot cover the great diversity of microbes. The ability to add reference spectra to the library in a routine laboratory is one of the great strengths of this mass-spectrometric identification method and should definitely be retained.

A second embodiment of a method according to the invention for the detection of microbe mixtures in the mass-spectrometric identification of a microbe sample with compilation of a score list is characterized by a procedure that, if two or more microbe species are present in the score list, the similarities between the reference spectra of the different microbe species are investigated and the microbe sample is indicated as a microbe mixture if the similarities are sufficiently low, below a specified similarity threshold. Here too, it is then possible to carry out the method of mixture analysis with combination spectra described above in order to identify the microbes which make up to the mixture.

In this second embodiment, the similarities between the reference spectra of the two or more microbe species in the score list are determined, and not the degree of relationship. This second embodiment of the invention is based on the finding that, in the end, the crucial factor is not the taxonomically determined relationships between the microbes, but only the similarity between the reference spectra. This also removes the complex problem with the microbe strains and their relationships. In order to exclude a mixture, a similarity threshold can be specified and set for the similarities between the reference spectra. This threshold is best determined experimentally, in studies with artificially produced mixtures, for example. Usually a mixture is present only when the similarities between the reference spectra of different microbe species are all below this specified similarity threshold. From these similarities it is even possible to deduce the probability of the presence of a mixture: the lower the similarity, the higher the probability of a mixture. If a mixture is detected by the method according to the invention, the microbe species involved must then be identified with the method of "mixture analysis with combination spectra" described above.

A third embodiment of a method according to the invention leads not only to the detection of microbe mixtures, but also immediately to the identification of the microbes involved in the mixture. As with the example embodiments above, a score list is compiled in the mass-spectrometric identification by carrying out similarity comparisons between the sample spectrum and all the reference spectra in a library. The further method comprises the following steps:
a) forming combination spectra from the reference spectra of different microbe species in the score list, b) determining the similarities between these combination spectra and the sample spectrum, c) selecting those combination spectra whose similarity to the sample spectrum is better than all the similarities between the sample spectrum and the pure reference spectra, d) computing the similarities between the combined reference spectra which make up this selected most similar combination spectra, e) indicating a mixture if the similarities of the combined reference spectra are below a specified similarity threshold, and if required f) identifying the microbes of the mixture as those whose combinations of reference spectra exhibit the best similarities to the sample spectrum.

Here also, optimum values for the specified similarity threshold in Step e) in this third embodiment are best determined experimentally.

This third embodiment of the method for recognizing mixtures initially uses the procedures of "mixture analysis with combination spectra" cited above, if several microbe species are present among the reference spectra of greatest similarity, but without already assuming firmly that a mixture is present. This may sound complicated and protracted, but it is not. The formation and similarity computation for around 100 combination spectra (it is usually considerably fewer) take only fractions of a second, a negligible time when compared to the similarity computations of the sample spectrum with the thousands of reference spectra in a library.

This third embodiment of mixture recognition is carried out in detail as follows: First, the identification method is carried out with computation of all the similarity scores between the sample spectrum and the reference spectra, and the score list of the most similar reference spectra is drawn up with microbe species and microbe strains. The score list does not have to be displayed on the screen; it is sufficient to collect it in the computer memory. The computer program then goes through the score list, starting with the reference spectrum with the greatest similarity. In general, several of the best similarities at the top of the list all belong to one microbe species if reference spectra of several strains of this microbe species are present in the library. If the program now hits on a second microbe species, combination spectra are formed from the first reference spectrum of this second microbe species together with all the reference spectra of the first microbe species, and they are each investigated regarding their similarity to the sample spectrum. The same method is used for further reference spectra of the second microbe species, and also for reference spectra of a third microbe species, if one is present. The reference spectra of the third microbe species are combined with all the reference spectra of the first and the second microbe species. If combination spectra are obtained whose similarities to the sample spectrum are greater than the best similarity from the score list, a mixture may be present, but this is not conclusive. To decide whether a mixture is to be indicated, the similarities between the two reference spectra making up each of these best combinations are checked. Only if they are below a specified similarity threshold, the computer program indicates to the user that this is most probably a mixture. On the basis of the degree of similarity between the reference spectra of the combinations, a statement can even be made on the level of probability that a mixture is present.

The invention thus proposes methods which allow an automatic evaluation program for the score lists to recognize the presence of a microbe mixture with a relatively high degree of certainty.

The proposed methods can, naturally, be changed and improved in a variety of ways. If, for example, a score list is always limited to the 20 or 30 most similar reference spectra (as is often the case), one can very frequently observe that the first five to 15 most similar reference spectra all belong to different strains of the same microbe species and (almost) all have high similarity scores, while there is a large gap in the similarity scores towards the next most similar reference spectra. It is obvious that in such unequivocal cases, a suitable computer program can indicate a definite identification of the species without having to check for the presence of a mixture. The presence of a mixture can therefore often be recognized from the structure of the similarity scores for the reference spectra in the score list. If the reference spectra are ordered according to similarities, as is usual, there are sometimes such large jumps in the differences between consecutive reference spectra that subsequent reference spectra can be disregarded. On the other hand, if the score list contains reference spectra of two or more different microbe species, none of which has a very high degree of similarity to the sample spectrum, this does not always have to indicate a mixture of microbe species. If the reference spectra of the different species are similar to each other, the two microbe species may be related to the microbe species of the sample, but no reference spectrum of this microbe species is present in the library. It is possible to integrate all these cases in the evaluation program by means of appropriately determined similarity thresholds.

## Claims

1. Method for the recognition of microbe mixtures in the mass-spectrometric identification of a microbe sample, with compilation of a score list of the reference spectra in a library which are most similar to the sample spectrum, **wherein** an exclusion list is provided in addition to the library, said exclusion list representing the relationship between microbe strains which have very similar mass spectra and are assigned to different microbe species, and, if two or more microbe species are present in the score list, the relationships between the microbe strains of different microbe species being present in the score list are determined with the aid of an exclusion list, and the microbe sample is indicated as a microbe mixture if the relationship is below a specified degree.

2. Method for the recognition of microbe mixtures in the mass-spectrometric identification of a microbe sample, with compilation of a score list of the reference spectra in a library which are similar to the sample spectrum, **wherein,** if two or more microbe species are present in the score list, the similarities between the reference spectra of different microbe species being present in the score list are investigated, and the microbe sample is indicated as a microbe mixture if the similarities are below a specified similarity threshold.

3. Method according to Claim 1 or 2, wherein, if a microbe mixture has been indicated, a mixture analysis using combination spectra is carried out.

4. Method for the detection and identification of microbe mixtures in the mass-spectrometric identification of a microbe sample, with compilation of a score list of the reference spectra in a library which are similar to the sample spectrum, **comprising the steps**
a) forming combination spectra from the reference spectra of the different microbe species in the score list,
b) computing the similarities between these combination spectra and the sample spectrum,
c) selecting the combination spectra whose similarity to the sample spectrum is better than the similarities between the sample spectrum and the pure reference spectra,
d) computing the similarities between the reference spectra combined for these selected combination spectra,
e) indicating a mixture if the similarities of the combined reference spectra are below a specified similarity threshold, and
f) indicating the microbe species of the mixture as those whose combinations of reference spectra demonstrate the best similarities to the sample spectrum.

5. Method according to one of the Claims 2 or 4, wherein the specified similarity thresholds are determined experimentally.

6. Method according to one of the Claims 1 to 5, wherein the reference spectra of the score list are differentiated from the other reference spectra in the library by a specified similarity threshold.

7. Method according to one of the Claims 1 to 5, wherein the score list contains a specified number of most similar reference spectra.

8. Method according to one of the Claims 1 to 5, wherein the score list contains all the reference spectra up to the reference spectrum where a specified jump takes place in the similarity score in relation to the next most similar reference spectrum.

## Patentansprüche

1. Verfahren zur Erkennung von Mikrobengemischen bei der massenspektrometrischen Identifizierung einer Mikrobenprobe mit Aufstellung einer Bestenliste mit den zum Probenspektrum ähnlichsten Referenzspektren einer Bibliothek, **dadurch gekennzeichnet, dass** zusätzlich zur Bibliothek eine Ausschlussliste bereitgestellt wird, wobei die besagte Ausschlussliste die Verwandtschaft zwischen Mikrobenstämmen darstellt, die sehr ähnliche Massenspektren besitzen und unterschiedlichen Mikrobenarten zugeordnet sind, und, bei Vorhandensein zweier oder mehrerer Mikrobenarten in der Bestenliste, die Verwandtschaften der Mikrobenstämme unterschiedlicher Mikrobenarten in der Bestenliste untereinander anhand einer Ausschlussliste festgestellt werden und die Mikrobenprobe als Mikrobengemisch ausgewiesen wird, falls die Verwandtschaft sich unterhalb eines festgesetzten Grads befindet.

2. Verfahren zur Erkennung von Mikrobengemischen bei der massenspektrometrischen Identifizierung einer Mikrobenprobe mit Aufstellung einer Bestenliste mit den zum Probenspektrum ähnlichen Referenzspektren einer Bibliothek, **dadurch gekennzeichnet, dass** bei Vorhandensein von zwei oder mehr gleichen Mikrobenarten in der Bestenliste die Ähnlichkeiten der Referenzspektren der verschiedenen Mikrobenarten, die sich in der Bestenliste befinden, untersucht werden und die Mikrobenprobe als Mikrobengemisch ausgewiesen wird, falls die Ähnlichkeiten sich unterhalb einer festgesetzten Ähnlichkeitsschwelle befinden.

3. Verfahren nach einem der Ansprüche 1 oder 2, **dadurch gekennzeichnet, dass** bei Ausweis eines Mikrobengemisches eine Gemischanalyse mithilfe von Kombinationsspektren durchgeführt wird.

4. Verfahren zur Erkennung und Identifizierung von Mikrobengemischen bei der massenspektrometrischen Identifizierung einer Mikrobenprobe mit Aufstellung einer Bestenliste mit den zum Probenspektrum ähnlichen Referenzspektren einer Bibliothek, **mit den Schritten**
a) Bildung von Kombinationsspektren aus den Referenzspektren jeweils verschiedener Mikrobenarten der Bestenliste,
b) Berechnung der Ähnlichkeiten dieser Kombinationsspektren zum Probenspektrum,
c) Ermittlung der Kombinationsspektren, deren Ähnlichkeit zum Probenspektrum höher ist als alle Ähnlichkeiten des Probenspektrums zu den reinen Referenzspektren,
d) Berechnung der Ähnlichkeiten zwischen den Referenzspektren, die jeweils für diese Kombinationsspektren kombiniert wurden,
e) Ausweisen eines Gemischs, wenn die Ähnlichkeiten der kombinierten Referenzspektren unterhalb einer festgelegten Ähnlichkeitsschwelle sind, und
f) Identifizierung der Mikrobenarten des Gemischs als diejenigen, deren Kombinationen von Referenzspektren die besten Ähnlichkeiten mit dem Probenspektrum aufweisen.

5. Verfahren nach einem der Ansprüche 2 oder 4, **dadurch gekennzeichnet, dass** die festgelegten Ähnlichkeitsschwellen experimentell ermittelt werden.

6. Verfahren nach einem der Ansprüche 1 bis 5, **dadurch gekennzeichnet, dass** die Referenzspektren der Bestenliste durch eine festgelegte Ähnlichkeitsschwelle von den übrigen Referenzspektren der Bibliothek abgegrenzt werden.

7. Verfahren nach einem der Ansprüche 1 bis 5, **dadurch gekennzeichnet, dass** die Bestenliste eine festgelegte Anzahl an ähnlichsten Referenzspektren enthält.

8. Verfahren nach einem der Ansprüche 1 bis 5, **dadurch gekennzeichnet, dass** die Bestenliste alle Referenzspektren bis zu demjenigen Referenzspektrum enthält, bei dem ein vorbestimmter Sprung im Ähnlichkeitswert zum nächstähnlichsten Referenzspektrum auftritt.

## Revendications

1. Méthode pour identifier des mélanges microbiens dans l'identification de spectrométrie de masse d'un échantillon microbien, avec génération d'une liste de résultat des spectres de référence d'une bibliothèque qui ressemblent le plus au spectre échantillon, **caractérisée en ce que** une liste d'exclusion est fournie en plus de la bibliothèque, ladite liste d'exclusion représentant la relation entre les souches microbiennes présentant des spectres de masse très similaires affectés à différentes variétés de microbes et, si deux variétés de microbes, ou plus, sont présentes dans la liste de résultat, **en ce que** les relations entre les souches microbiennes de différentes variétés de microbes présentes dans la liste de résultat sont déterminées au moyen d'une liste d'exclusion et que l'échantillon de microbes est déclaré mélange microbien si la relation est inférieure à un degré déterminé.

2. Méthode pour identifier des mélanges microbiens dans l'identification de spectrométrie de masse d'un échantillon microbien, avec génération d'une liste de résultat des spectres de référence d'une bibliothèque qui sont similaires au spectre échantillon, **caractérisée en ce que,** si deux variétés de microbes, ou plus, sont présentes dans la liste de résultat, les similarités entre les spectres de référence de différentes variétés de microbes présentes dans la liste de résultat sont recherchées et que l'échantillon de microbes est déclaré mélange microbien si les similarités sont inférieures à un seuil de similarité déterminé.

3. Méthode selon la revendication 1 ou 2, **caractérisée en ce que**, si un mélange microbien a été déclaré, une analyse du mélange est effectuée au moyen de spectres combinés.

4. Méthode pour détecter et identifier des mélanges microbiens dans l'identification de spectrométrie de masse d'un échantillon de microbes, avec génération d'une liste de résultat des spectres de référence d'une bibliothèque qui sont similaires au spectre échantillon, **comprenant les étapes suivantes** :
a) Formation de spectres combinés à partir des spectres de référence des différentes variétés de microbes dans la liste de résultat ;
b) Calcul des similarités entre ces spectres combinés et le spectre échantillon ;
c) Sélection des spectres combinés dont la similarité avec le spectre échantillon est meilleure que les similarités entre le spectre échantillon et les spectres de pure référence ;
d) Calcul des similarités entre les spectres de référence combinés pour lesdits spectres combinés sélectionnés ;
e) Indication d'un mélange si les similarités des spectres de référence combinés sont inférieures à un seuil de similarité déterminé, et
f) Indication des variétés de microbes du mélange comme celles dont les spectres de référence combinés présentent les meilleurs similarités avec le spectre échantillon.

5. Méthode selon l'une des revendications 2 ou 4, **caractérisée en ce que** les seuils de similarité déterminés sont définis de manière expérimentale.

6. Méthode selon l'une des revendications 1 à 5, **caractérisée en ce que** les spectres de référence de la liste de résultat sont différenciés des autres spectres de référence dans la bibliothèque par un seuil de similarité déterminé.

7. Méthode selon l'une des revendications 1 à 5, **caractérisée en ce que** la liste de résultat contient un nombre déterminé de spectres de référence les plus similaires.

8. Méthode selon l'une des revendications 1 à 5, **caractérisée en ce que** la liste de résultat contient tous les spectres de référence jusqu'au spectre de référence pour lequel le résultat de similarité fait un saut déterminé par rapport au spectre de référence le plus similaire suivant.
